# EUROPEAN PATENT SPECIFICATION

(11) **EP 3 706 764 B1**
(45) Date of publication and mention of the grant of the patent: **06.12.2023**
(21) Application number: 18792405.5
(22) Date of filing: 29.10.2018
(51) Int. Cl.: A61K 31/736, A61K 36/28, A61K 36/68, A61P 1/10

(54) **COMPOSITION FOR THE ACUTE AND CHRONIC TREATMENT OF CONSTIPATION**
ZUSAMMENSETZUNG ZUR AKUTEN UND CHRONISCHEN BEHANDLUNG VON VERSTOPFUNG
COMPOSITION DESTINÉE AU TRAITEMENT AIGU ET CHRONIQUE DE LA CONSTIPATION

(30) Priority: 10.11.2017 IT 201700128680
(43) Date of publication of application: 16.09.2020
(73) Proprietor: Neilos S.r.l., 80063 Piano di Sorrento (NA) (IT)
(72) Inventor: DI MAIO, Umberto, 80063 Piano di Sorrento NA (IT)
(74) Representative: Longoni, Alessandra
(86) International application number: PCT/EP2018/079570
(87) International publication number: WO 2019/091812

(56) References cited:
- US-A1- 2009 304 831
- Ema - European Medicines Agency: "Assessment report on Plantago ovata Forssk., seminis tegumentum", , 14 March 2013 (2013-03-14), XP055476432, Retrieved from the Internet: URL:http://www.ema.europa.eu/docs/en_GB/do cument_library/Herbal_-_HMPC_assessment_re port/2013/07/WC500146506.pdf [retrieved on 2018-05-17]
- Maria Elena Frosini: "Stitichezza: ecco i rimedi naturali", , 4 May 2013 (2013-05-04), XP055476561, Retrieved from the Internet: URL:http://www.rodiola.info/stiticheza-rim edi-naturali.php [retrieved on 2018-05-18]
- V. LOENING-BAUCKE ET AL: "Fiber (Glucomannan) Is Beneficial in the Treatment of Childhood Constipation", PEDIATRICS, vol. 113, no. 3, 1 March 2004 (2004-03-01) , pages e259-e264, XP055476563, ISSN: 0031-4005, DOI: 10.1542/peds.113.3.e259

## Description

The present invention refers to a composition containing an association of the extract of *Plantago ovata* or *Plantago psyllium,* glucomannan and an extract of *Matricaria chamomilla* for the acute and chronic treatment of constipation. The composition is particularly effective thanks to the synergistic action of its components.

### Background of the invention

Constipation is a common gastrointestinal disorder which affects about 28% of the Western world population. Epidemiological studies demonstrate that this problem is mainly encountered in elderly, children and women and it deeply interferes with the life quality of said subjects.

In recent years several scientific studies have focused on researching an adequate definition for the term constipation on the basis of some parameters such as faeces weight and consistency, time of colonic transit and evacuation difficulties.

The most important definitions refer to alterations of the above-mentioned parameters and constipation is mainly defined as a temporary or chronic condition wherein there is a delayed transit of faeces in the intestine entailing infrequent, difficult or incomplete evacuations. This condition can cause pain and abdominal distention as well as anorexia and vomit and other less specific symptoms such as headache, halitosis, confusion, etc.

The causes of constipation are mainly three: lifestyle (functional constipation), secondary condition of other diseases or induced by the use of some drugs. According to the most valid criteria, functional constipation is defined and diagnosed as the presence of symptoms for three months with an onset thereof for at least six months. The most specific diagnostic criteria are: straining during at least 25% of evacuations, hard and granulose faeces for at least 25% of evacuations, sensation of incomplete evacuation for at least 25% of evacuations, sensation of anal blockage or obstruction for at least 25% of evacuations, the use of manual maneuvers to facilitate at least 25% of evacuations, fewer than three evacuations per week and rarely loss of faeces. The physiopathology of functional constipation, however, remains still not well clear but it is believed to be mainly due to a slow colonic transit and/or pelvic floor dysfunctions. As regards the colonic transit, this slowing can be due to either excessive segmental contractions or a reduced contraction of distal colon.

As mentioned before, the constipation may also be due to the use of some drugs and in this case it is referred to as iatrogenic constipation. Primarily, opioid analgesics can induce alterations of the intestinal functionality through their action on the central nervous system and mainly on the opioid receptors present at enteric level. Also anticholinergic drugs can induce constipation by blocking the acetylcholine receptors present at intestinal level. Also tricyclic antidepressants show the same mechanism as anticholinergics in potentially causing constipation. Diuretics, by reducing the absorption of water during the formation of faeces or inhibiting the secretion, reduce intestinal motility and can lead to hypokalemia and constipation. Still other drugs that can cause constipation are antiparkinsonian agents, antihypertensives, MAGs inhibitors, antipsychotics, cholestyramine, etc.

Treatments usually performed in subjects suffering from this disorder vary according to the entity and cause of constipation. Initially, a conservative treatment comprising changes in diet and lifestyle, among which mostly a greater consumption of fibres, is preferred. In particular, in this regard reference is made to a consumption of about 30 g per day to ensures beneficial effects and prevent collateral events such as abdominal pain, flatulence and diarrhoea.

Another common practice is the so-called biofeedback which can be useful in effectively coordinating the pelvic floor distention and the anal sphincter relaxation in order to facilitate the passage of faeces.

When a simple variation in eating habits is not enough, pharmacological therapy is used which involves: bulk-forming laxatives and stool softeners, stimulant laxatives, osmotic laxatives, prokinetics and in the most serious cases enemas.

Bulk-forming laxatives have the same effect of fibres and include for example methylcellulose, psyllium and sterculia. These agents absorb water and increase the faecal mass promoting the frequency and consistency of faeces.

Docusate instead is the main agent used to soften faeces and its mechanism is linked to the ability to lower the surface tension of faeces facilitating the entry of water. Stimulant laxatives instead include plants containing anthraquinones (senna, cascara, aloe, frangula, rhubarb, etc.), diphenylamine derivatives (bisacodyl) and sodium picosulfate.

The use thereof is very common in both acute and chronic constipation and is linked to the ability to increase the intestinal motility and the secretion of water and electrolytes. Sodium picosulfate is mainly used for the preparation to endoscopy or other type of analysis.

Osmotic laxatives include magnesium and salts thereof, poorly absorbable sugars and polyethylene glycol-based preparations (PEG). These actives should represent the first line treatment to be followed in case the treatment with fibres or other changes in the lifestyle did not give the expected results. The mechanism of action thereof is linked to their ability to reach the colon in an unchanged manner establishing an osmotic gradient which increases faecal volume and peristalsis.

Prokinetic drugs are mainly cholinergic or serotonin 5-HT4 receptor agonists or of another type such as erythromycin, domperidone and metoclopramide.

The cholinergic agonists which are mostly recommended are bethanechol (25-50 mg 3 to 4 times a day reduces the constipation induced by tricyclic antidepressants) and neostigmine even if there are no significant scientific studies in support.

Among the 5-HT4 receptor agonists the main one is prucalopride which is recommended if the use of other types of laxatives has not brought about the desired effect. The use at a dosage between 1 and 2 mg increases the intestinal motility and decreases the time of colonic transit.US2009304831 discloses a laxative composition based an anthranoid laxatives and including other components, inter alia, glucomannan powder, Psyllium powder and a chamomile extract.

Now it has been surprisingly found that an association of an extract of *Plantago ovata* or *Plangato psyllium,* glucomannan and an extract of *Matricaria chamomilla* is particularly effective in the acute and chronic treatment of constipation thanks to the synergistic actions of its components.

### Detailed description of the invention

Therefore an object of the present invention is a composition containing an association of an extract of *Plantago ovata* or *Plangato psyllium,* glucomannan and an extract of *Matricaria chamomilla* useful for the acute and chronic treatment of constipation.

The components of the association according to the present invention are all known components, widely used in therapy.

### Extract of a plant belonging to the genus Plantago (Psyllium)

With the term "psyllium" are commonly identified the plants or the seed of different plants belonging to the genus *Plantago* and to the family of Plantaginaceae.

This genus comprises numerous plant species (for example *Plantago arenaria, Plantago indica, Plantago afra, Plantago lanceolata, Plantago major, Plantago media)* and in particular, those more recognised and cultivated are the species of *Plantagoovata* ("ispaghula") and *Plantago psyllium* to which reference is usually made when speaking of psyllium.

The functional part of the plant is represented by the seeds from which the tegument enveloping them is obtained which is commonly identified with the name of cuticle (see, for example, Assessment report on Plantago ovata Forssk., by EMA).

The psyllium peel contains large amounts of hemicellulose, while the seed is composed of 35% of soluble fibres and of 65% of insoluble polysaccharides. The psyllium is considered as a mucilaginous fibre because it is able to form a gel in aqueous solution. This ability is related to its role within the endosperm of the plant seeds, where it stores water preventing the drying.

To the psyllium are ascribed several properties potentially useful for treating constipation: said fibre is able to increase the weight of the faecal mass and to favour the production of short chain fatty acids (SCFA) particularly important for the intestine wellbeing. The increase of the faecal mass as well as of the faeces hydration is ascribable to the fibre ability to swallow in contact with biological fluids and to reach the intestinal caecum in an intact and highly polymerised form. Moreover, by partial fermentation of psyllium fibres SCFA are produced among which acetate, propionate and butyrate. Butyric acid is particularly important for the enterocytes and from scientific studies it seems potentially valuable in cases of colon-rectal cancer and ulcerative colitis.

For these properties the psyllium has been evaluated as a valid remedy in cases of constipation, faecal incontinence, haemorrhoids, ulcerative colitis, to reduce appetite, for dyslipidaemias and for diabetes mellitus.

Its main use however remains relative to constipation about which several clinical studies have been carried out.

In particular, in a study carried out in 1997 by Voderholzer and collaborators the consumption of 15-30 g of psyllium per day assured an improvement or a resolution of the symptomatology in patients suffering from chronic constipation after a period of 6 weeks. The study highlights how the psyllium is particularly effective where there is not a clear pathological condition inducing constipation while it is resulted less effective in patients with slow colonic transit or in patients with severe disorders such as rectocele, internal prolapses, rectal hyposensitivity or anismus.

Also in comparative studies psyllium has shown good results in terms of efficacy, for example in a comparative study with docusate sodium (100 mg 2 times per day) psyllium (5.1 g two times per day) has shown to be superior in patients with idiopathic chronic constipation. In particular the study has demonstarted that patients treated with psyllium-based preparation showed a higher water content in the faeces and a general improvement of the constipation symptoms which was the more significant as increasing the treatment time (treatment duration in the study 2 weeks).

In the light of the wide use in commercial product and on the basis of the good scientific documents, a plant extract belonging to the genus *Plantago* can be considered a valid component for the prevention, treatment or improvement of constipation.

### Glucomannan

Glucomannan is a neutral polysaccharide produced by several plants where it plays a role of energy supply and in some cases of structural support. This polysaccharide is mainly made up of mannose residues with glucose as secondary sugar and it can contain acetylated residues and galactose in lateral chains. The characteristics in terms of molecular weight and mannose/glucose ratio of glucomannan vary depending on plant type and extraction method. Primarily the most used source is glucomannan obtained by the bulb of *Amorphophallus konjac* which is a plant belonging to the Araceae family. The flour obtained from the bulb is conventionally used as gelling agent and thickener and it is also allowed in the European regulations as food ingredient (E425). Glucomannan in fact has a high ability to swell when hydrated.

These compounds such as glucomannan are usually poorly absorbed at intestinal level and can represent an interesting source of fibre which favours the intestinal equilibrium. Glucomannan is a soluble fibre that unlike others such as pectin and guar gum can be consumed in a lower amount and has not a particular smell or taste. The mechanism of action of glucomannan is due to its ability to absorb large amounts of water increasing intestinal movements and faecal mass moreover at the same time is able to improve and maintain at its best the intestinal flora enhancing the growth of lactobacilli and bifidobacteria and inhibiting the growth of harmful microorganisms such as Clostridium.

Several clinical studies evaluated the efficacy of the consumption of glucomannan in children and adults verifying its ability to improve the symptomatology of patients suffering from constipation.

The largest study is a multicentre study carried out in Italy in 1991 which involved 93 patients with chronic constipation. The study was subdivided in an initial phase which provided for the assumption of 1 g of glucomannan three times a day for a month, and a maintenance phase which provided for the consumption of 1 g of glucomannan two times per day for another month. After the first month all patients registered a statistically significant improvement for all the observed parameters (number of intestinal movements per week and number of enemas). Glucomannan have proved to be well tolerated without significant side effects.

Another study published in 2013 on 7 patients with a low fibre diet provided for a 21 days period with placebo, a 7 days period of adaptation and 21 days of glucomannan supplementation (1.5 g three times a day). Supplementation with glucomannan has proved to be able to increase the weekly defecation frequency from 4.1 ± 0.6 to 5.3 ± 0.6 (30 %) favouring the intestinal movement. Moreover the supplement increased the amount of lactobacilli and bifidobacteria in faeces thereby reducing the amount of clostridia.

In literature are also present some articles relative to the administration of glucomannan in children.

In particular in an article published on *Pediatrics* in 2004 the consumption of 100 mg/kg of the active ingredient (maximum 5 g a day) improved the intestinal movements frequency, faeces consistency, abdominal pain and in general the treatment of constipation compared to placebo.

Although further studies in support are needed, glucomannan seems to be a valuable alternative to other types of fibres to be used as active ingredient within a formulation for the treatment of constipation symptomatology thanks to its ability to absorb large amounts of water and at the same time, after bacterial fermentation, favour the colonic pH and the proliferation of bacteria beneficial to our organism.

### Camomile

With the term camomile, reference is usually made to two different genera of plants such as *Chamaemelum* and *Matricaria,* In particular, the species *Chamaemelum nobile* (*Anthemis nobilis*) is identified as Roman camomile and the specie *Matricaria chamomilla* o *Matricaria recutitia* is known as German camomile. Camomile represents one of the most known plants and has been used for thousands of years. Botanically camomile is an annual plant belonging to the *Asteraceae* or *Compositae* family with thin roots penetrating the soil. The stem is hardly branched and grows between 10 and 80 cm. Leaves are long and narrow and the flowers are combined in small flower heads with a characterizing very pleasant smell.

Camomile represents a very important source of bioactive compounds belonging to the sesquiterpenes, flavonoids, coumarins and polyacetylenes classes. Cumarins which are present are umbelliferon and herniarin. Flavonoids which are contained in higher concentration are apigenin, luteolin and quercetin while the most abundant sesquiterpenes are chamazulene and alpha bisabolol.

To camomile and in particular to the flower extracts were ascribed several beneficial properties such as anti-inflammatory, spasmolytic, antioxidant activity, protective role in diseases at respiratory and gastrointestinal level. In particular the flavonoid components thereof and sesquiterpene compounds are the main responsible of said effects.

Relative to the present invention camomile is particularly suitable in the treatment of mild intestinal disorders such as bloating, flatulence, digestion difficulties, gastrointestinal irritations, spasms often occurring in people with acute or chronic constipation or also following the use of some drugs for constipation that can provoke them.

This effect is mediated by the anti-inflammatory, carminative and anti-spasmodic action of its components mainly apigenin and other flavonoids. Moreover, has been reported in the literature a potential action of apigenin at level of GABA receptors may contribute to a mild sedative placating effect. Also other substances such as umbelliferon or spiroethers show to have antispasmodic activity and thus relaxing. Just as reported in the EMA monograph about camomile flowers, the indication relative to the use for mild gastrointestinal disorders is linkable to the wide traditional use and also to a clinical study carried out on patients suffering from gastrointestinal disorders of varying extents.

Moreover the potential effectiveness of camomile in facilitating evacuations in persons suffering from constipation was evaluated in another clinical study on patients with irritable bowel. In this case, the consumption of a camomile based product for 4 weeks assured normal defecation in 80% of the patients with improvement in faeces consistency.

The potential beneficial action of camomile within a product for the maintenance in case of chronic constipation or in case of a low-fibre diet, can be justified by its coadjutant action in disorders that can be coexistent with constipation such as bloating, flatulence or digestive problems of varying extents. Also the presence of mucilages may be particularly useful to soothe digestive apparatus walls.

Moreover, the good safety profile of camomile extracts makes them well suited also for the administration to children and pregnant women.

In a preferred embodiment, the composition object of the present invention contain the association of the extract of *Plantago ovata* or *Plantago psyllium,* glucomannan and an extract of *Matricaria chamomilla* in admixture with a suitable acceptable carrier.

Suitable acceptable carriers are those commonly known to the man skilled in the art for the preparation of compositions for oral administration such as solutions, suspensions, powders or granulates, tablets, capsules, pellets. By way of non-limiting example, said acceptable carriers can consists of binders, diluents, lubricants, glidants, disintegrants, solubilizing (wetting) agents, stabilizers, colorants, anti-caking agents, emulsifiers, thickeners and gelling agents, coating agents, humectants, sequestrants, and sweeteners.

Specifically examples of diluents can be: magnesium carbonate, cellulose microcrystalline, starch, lactose, and sucrose; mainly used lubricants are magnesium stearate, stearic acid, and sodium stearyl fumarate. As glidants colloidal silica and magnesium silicate, as disintegrants the cross-linked polyvinylpyrrolidones and sodium starch glycolate, as solubilizing agents surfactants such as TWEEN or sodium lauryl sulphate, and as stabilizers all classes of preservatives (sorbic acid and derivatives, benzoic acid and derivatives, parabens), antioxidants (ascorbic acid and derivatives, tocopherol), and acidifying agents (phosphoric acid, tartaric acid) can be mentioned. Thickeners and gelling agents can be carrageenan, pectins and starches, coating agents include for example waxes and derivatives, anti-caking agents include for example calcium or magnesium carbonate, humectants include for example sorbitol and mannitol, sequestrants include for example EDTA and derivatives, sweeteners include for example aspartame and acesulfame potassium.

The composition object of the present invention is preferably a liquid or solid composition for oral use, even more preferably an aqueous solution, a suspension, a powder or granulate, a tablet, a capsule, a pellet.

The composition object of the present invention can be a medical device, a food supplement, a nutraceutical, dietetic and nutritional composition, a food product, a beverage, a nutraceutical product, a medicament, a medicated food, a pharmaceutical composition or a food for special medical purposes.

The composition object of the present invention contains the extract of *Plantago ovata* or *Plantago psyllium,* glucomannan and an extract of *Matricaria chamomilla.*

The extract of *Plantago ovata* or *Plantago psyllium* is preferably present in an amount between 50 mg and 10 g, preferably between 100 mg and 7000 mg, still more preferably between 200 mg and 5000 mg.

Glucomannan is present in an amount between 50 mg and 10 g, preferably between 100 mg and 7000 mg, still more preferably between 200 mg and 5000 mg.

The extract of *Matricaria chamomilla* is present in an amount between 1 mg and 2000 mg, preferably between 10 mg and 1000 mg, still more preferably between 30 mg and 700 mg.

The compositions object of the present invention are particularly effective in the acute or chronic treatment of constipation allowing to obtain at the same time the regularity of the intestinal transit, increase of faeces weight and consistency, decrease of symptoms such as bloating, flatulence and spasms, and the achievement of a general intestinal wellbeing thanks to the synergistic action of their components.

Therefore a further object of the present invention is a composition containing the association of the extract of *Plantago ovata* or *Plantago psyllium,* glucomannan and an extract of *Matricaria chamomilla* in admixture with a suitable acceptable carrier for use in the acute and chronic treatment of constipation.

Without being bound to a specific theory, the inventors are of the opinion that the synergistic effect of the association of the extract of *Plantago ovata* or *Plantago psyllium,* glucomannan and an extract of *Matricaria chamomilla* according to the present invention, derives from the following activities of the components of the association.

The extract of *Plantago,* swelling in contact with biological fluids, is able to increase weight and to improve consistency of faeces. Moreover it favours the development of butyric acid particularly important for the enterocytes wellbeing.

Glucomannan is a soluble fibre odourless and tasteless which favours the evacuation and enhances the proliferation of lactobacilli and bifidobacteria contrasting the development of harmful bacteria.

Camomile, thanks to its numerous components, is a valuable coadjutant to eliminate symptoms such as bloating, flatulence, digestive difficulties and spasms further exerting a lenitive effect on digestive system mucosae.

The efficacy of the composition object of the present invention is evaluated with the following experimental protocol.

Constipation is induced through the administration to animals (mice and rats, preferably mouse) of compounds such as loperamide (3 mg/Kg) which binds to µ, κ and δ receptors leading to a reduction of muscle contractility at intestinal level and a reduction of secretions. Moreover said drug increases the absorption of water and reduces the contraction of sphincters reducing the evacuation frequency. Also other compounds that induce this type of effects or variation in diet (such as diets rich in fibres or with a high protein content) can anyway be used as experimental model of constipation.

After the induction of constipation, the compositions according to the present invention and the comparative compositions are orally administered to animals at established dosages comparing them with a control group normally administered with a saline solution or however an inert substance. To evaluate the potential laxative effect, the feces of animals are collected every day for a variable period of time. From the excreted feces, the number, the consistency in terms of weight and the water content (%), which is obtained from the ratio between wet feces weight minus dried feces weight divided by wet feces weight all multiplied by one hundred, are determined.

Another test that can be performed to assess the potential laxative action in terms of increase of intestinal motility is the study of the intestinal transit that is usually performed through the administration of coal or other similar compounds to animals after loperamide administration. Successively to constipation induction, animals are maintained without food in cages with wide meshes to facilitate feces fall.

After the administration (preferably after 30 minutes) of the compositions according to the present invention, or of the comparative compositions, animals are administered with a suspension (containing agents such as methylcellulose or preferably gum arabic) of coal (for example at 3-10%). After about 20-30 minutes the animals are sacrificed and the percentage of coal transit is obtained from the following equation: total length of small intestine - distance covered by the coal/total length of small intestine *100

Moreover, the synergistic effect of the composition can be highlighted evaluating the propulsion in the distal colon after six days from loperamide administration. Thirty minutes after samples administration (or the control and the vehicle), a glass pearl of 3 mm is inserted through the anus in the distal colon for 2 cm. For each animal the medium expulsion time (MET) of the glass pearl will be evaluated; an high MET value indicates an elevated reduction of the intestinal motility.

Using the above-described experimental protocols, the laxative action of the association of the extract of *Plantago ovata* or *Plantago psyllium,* glucomannan and an extract of *Matriacaria chamomilla,* according to the present invention, and of the individual components of the association is evaluated to verify the synergistic effect.

### Examples

By way of example are now provided some non-binding examples of daily doses of active components of the compositions object of the present invention.

Daily doses are meant to be administered in a suitable oral dosage form and divided in one or more dosage units.

### EXAMPLE 1 (powder or granulate for oral use)

| Active ingredient | Amount for daily dose |
|---|---|
| *Plantago ovata* | 4 g |
| Glucomannan | 2 g |
| *Matricaria chamomilla* | 100 mg |

### EXAMPLE 2 (powder or granulate for oral use)

| Active ingredient | Amount for daily dose |
|---|---|
| *Plantago psyllium* | 4 g |
| Glucomannan | 1 g |
| *Matricaria chamomilla* | 200 mg |

### EXAMPLE 3 (powder or granulate for oral use)

| Active ingredient | Amount for daily dose |
|---|---|
| *Plantago ovata* | 3.5 g |
| Glucomannan | 1.5 g |
| *Matricaria chamomilla* | 300 mg |

Oral formulations are prepared by conventional techniques such as mixing.

## Claims

1. A composition consisting of an extract of *Plantago ovata* or *Plantago psyllium,* glucomannan and an extract of *Matricaria chamomilla* in admixture with a suitable acceptable carrier.

2. A composition according to claim 1 in the form of a liquid or solid composition for oral use.

3. A composition according to claim 2 in the form of aqueous solution, suspension, powder or granulate, capsule, tablet, pellet.

4. A composition according to any of the previous claims wherein the extract of *Plantago ovata* or *Plantago psyllium* is present in an amount between 50 mg and 10 g, preferably between 100 mg and 7000 mg, still more preferably between 200 mg and 5000 mg, glucomannan is present in an amount between 50 mg and 10 g, preferably between 100 mg and 7000 mg, still more preferably between 200 mg and 5000 mg, and the extract of *Matricaria chamomilla* is present in an amount between 1 mg and 2000 g, preferably between 10 mg and 1000 mg, still more preferably between 30 mg and 700 mg.

5. Composition according to any of the previous claims wherein the composition is a medical device, a food supplement, a nutraceutical, dietetic and nutritional composition, a food product, a beverage, a nutraceutical product, a medicament, a medicated food, a pharmaceutical composition or a food for special medical purposes.

6. Composition according to any of the previous claims for use in the acute and chronic treatment of constipation.

## Patentansprüche

1. Zusammensetzung, die aus einem Extrakt von *Plantago ovata* oder *Plantago psyllium,* Glucomannan und einem Extrakt von *Matricaria chamomilla* in Gemisch mit einem geeigneten annehmbaren Träger besteht.

2. Zusammensetzung nach Anspruch 1 in Form einer flüssigen oder festen Zusammensetzung zur oralen Verwendung.

3. Zusammensetzung nach Anspruch 2 in Form einer wässrigen Lösung, Suspension, eines Pulvers oder Granulats, einer Kapsel, Tablette oder eines Pellets.

4. Zusammensetzung nach einem der vorherigen Ansprüche, wobei der Extrakt von *Plantago ovata* oder *Plantago psyllium* in einer Menge zwischen 50 mg und 10 g, vorzugsweise zwischen 100 mg und 7000 mg, noch bevorzugter zwischen 200 mg und 5000 mg vorhanden ist, Glucomannan in einer Menge zwischen 50 mg und 10 g, vorzugsweise zwischen 100 mg und 7000 mg, noch bevorzugter zwischen 200 mg und 5000 mg vorhanden ist, und der Extrakt von *Matricaria chamomilla* in einer Menge zwischen 1 mg und 2000 g, vorzugsweise zwischen 10 mg und 1000 mg, noch bevorzugter zwischen 30 mg und 700 mg vorhanden ist.

5. Zusammensetzung nach einem der vorherigen Ansprüche, wobei die Zusammensetzung ein Medizinprodukt, ein Nahrungsergänzungsmittel, ein Nahrungsergänzungsmittel, eine diätetische und ernährungsphysiologische Zusammensetzung, ein Lebensmittelprodukt, ein Getränk, ein nutrazeutisches Produkt, ein Medikament, ein medizinisches Lebensmittel, eine pharmazeutische Zusammensetzung oder ein Lebensmittel für spezielle medizinische Zwecke ist.

6. Zusammensetzung nach einem der vorherigen Ansprüche zur Verwendung bei der akuten und chronischen Behandlung von Verstopfung.

## Revendications

1. Composition constituée d'un extrait de *Plantago ovata* ou de *Plantago psyllium,* de glucomannane et d'un extrait de *Matricaria chamomilla* en mélange avec un vecteur acceptable approprié.

2. Composition selon la revendication 1 sous la forme d'une composition liquide ou solide à usage oral.

3. Composition selon la revendication 2 sous la forme d'une solution aqueuse, d'une suspension, d'une poudre ou d'un granulé, d'une capsule, d'un comprimé, d'une pastille.

4. Composition selon l'une quelconque des revendications précédentes, ledit extrait de *Plantago ovata* ou de *Plantago psyllium* étant présent en une quantité comprise entre 50 mg et 10 g, de préférence entre 100 mg et 7000 mg, encore plus préférablement entre 200 mg et 5000 mg, ledit glucomannane étant présent en une quantité comprise entre 50 mg et 10 g, de préférence entre 100 mg et 7000 mg, encore plus préférablement entre 200 mg et 5000 mg, et ledit extrait de *Matricaria chamomilla* étant présent en une quantité comprise entre 1 mg et 2000 g, de préférence entre 10 mg et 1000 mg, encore plus préférablement entre 30 mg et 700 mg.

5. Composition selon l'une quelconque des revendications précédentes, ladite composition étant un dispositif médical, un complément alimentaire, une composition nutraceutique, diététique et nutritionnelle, un produit alimentaire, une boisson, un produit nutraceutique, un médicament, un aliment médicamenteux, une composition pharmaceutique ou un aliment destiné à des fins médicales spéciales.

6. Composition selon l'une quelconque des revendications précédentes destinée à être utilisée dans le traitement aigu et chronique de la constipation.
